# EUROPEAN PATENT SPECIFICATION

(11) **EP 4 547 360 B1**
(45) Date of publication and mention of the grant of the patent: **04.11.2026**
(21) Application number: 23734681.2
(22) Date of filing: 28.06.2023
(51) Int. Cl.: B01D 15/22, B01D 63/08, C07K 1/34, B01D 61/14, B01D 15/08, B01D 71/08, B01D 69/14, C07K 1/16, C07K 1/36

(54) **TANGENTIAL FLOW FILTRATION/CHROMATOGRAPHY SYSTEMS, USE THEREOF, AND METHODS OF SEPARATION**
TANGENTIALFLUSS-FILTRATIONS/CHROMATOGRAFIE-SYSTEME, VERWENDUNG DAVON UND VERFAHREN ZUR TRENNUNG
SYSTÈMES DE FILTRATION/CHROMATOGRAPHIE À FLUX TANGENTIEL, LEUR UTILISATION ET PROCÉDÉS DE SÉPARATION

(30) Priority: 29.06.2022 EP 22181999
(43) Date of publication of application: 07.05.2025
(73) Proprietor: Sartorius Stedim Biotech GmbH, 37079 Göttingen (DE)
(72) Inventor: DIEL, Bernhard, 37079 Göttingen (DE); THOM, Volkmar, 37079 Göttingen (DE); PETERSEN, Rebecca, 37079 Göttingen (DE)
(74) Representative: Novagraaf International SA
(86) International application number: PCT/EP2023/067658
(87) International publication number: WO 2024/003141

(56) References cited:
- EP-A1- 4 019 125
- US-A1- 2005 197 496
- US-A1- 2010 059 443
- US-A1- 2017 173 537
- US-A1- 2020 368 696
- US-A1- 2021 284 687

## Description

The present invention relates to tangential flow filtration/chromatography systems, the use thereof, and methods of separation, particularly simultaneously separating one or more target molecules from cells, cell debris and further contaminants contained in e.g. fermentation broth or any other particulate and impurity containing, non-clarified liquid.

In the biopharmaceutical industry, there is a continuing trend towards increased efficiency and intensification of cultivation processes. Constantly improving technologies and optimized processes lead, e.g. in mAb-based products and processes, to ever smaller fermentation volumes with simultaneously increasing cell and protein concentrations, which must be processed in the further process steps for clarification and isolation of the actual target product. More and more frequently, the cultivation is generated in fed-batch, high density fed-batch or as a perfusion process, which means additional new challenges for the cell separation step.

Particularly the first clarification step of cell separation directly after cultivation is therefore becoming increasingly important, since high target product concentrations are usually also associated with high cell numbers, and thus with increasingly large bio-moisture masses (solid content, biomass). Conventional methods for cell separation, such as centrifugation and depth filtration or even tangential flow filtration (TFF) processes, often reach technical or even economic limits. In addition, the biopharmaceutical industry is showing a steadily increasing interest in single-use process solutions, with the aim of optimizing and accelerating costly and time-consuming validation processes of stainless steel systems, at all intermediate stages on the way to developing a new drug.

The acceptance of single-use (SU) technologies throughout the biopharmaceutical manufacturing process means that new and innovative technologies and products are constantly being sought to address these challenges.

Cell separation in SU production plants is classically accomplished by centrifugation followed by two or more depth filtration steps. Traditionally, however, this filtration technology is limited in terms of the concentration of the cell masses to be separated, since rapid blocking of the filter surface can hardly be prevented. At the same time, there are currently no hermetically sealed SU separation steps as users would like to use in order to exclude product contact with the environment or even with the operating personnel.

Therefore, technologies are permanently searched for, which make it possible to combine one or more clarification steps, to simplify them, or to make the first cell separation step more efficient and cheaper, or even to eliminate it completely.

In general, the first clarification step, in which the coarse particulate and colloidal constituents such as cells, cell components as well as other undesirable constituents are removed, can be seen as mandatory in the majority of cases. The aim here is to remove substances that make the classic process steps in the downstream inefficient or even impossible. Currently, the solid-liquid separation and clarification of the reactor solution in bioprocess engineering is therefore regarded as an inherent and central component which, according to general understanding, cannot be eliminated in order to be able to carry out the further separation steps in the first place.

In view of the above, the technical problem underlying the present invention is to provide systems and methods for simultaneously separating one or more target molecules from cells, cell debris and further contaminants contained in fermentation broth or any other particulate and impurity containing, non-clarified liquid, the systems and methods allowing to partially or completely eliminate classical clarification steps such as centrifugation, depth filtration, and also sterile filtration before chromatography.

The solution to the above technical problem is achieved by providing the embodiments characterized in the claims.

In particular, there is provided a tangential flow filtration/chromatography system for simultaneously separating one or more target molecules from cells, cell debris and further contaminants, the flow filtration/chromatography system comprising
a tangential flow filtration/chromatography device comprising at least one fluid inlet, at least one retentate outlet, at least one permeate outlet, and at least one membrane which separates a retentate section from a permeate section in the tangential flow filtration/chromatography device, the at least one retentate outlet being in the retentate section and the least one permeate outlet being in the permeate section,
wherein a flow from the at least one fluid inlet to the at least one retentate outlet is guided in the tangential flow filtration/chromatography device tangentially along the surface of the at least one membrane,
wherein the at least one membrane has chromatographically active centers for selectively binding the one or more target molecules or for selectively binding the further contaminants, and has convective pores which are flow through macropores providing flow passages or channels through the membrane and having a specific convective pore size;
wherein the at least one membrane comprises a polymer network material-based self-supporting bi-continuous separation matrix comprising a first continuous phase and a second continuous phase, the at least one membrane optionally comprising a third inert phase, wherein the first continuous phase is a portion of the matrix which is formed by a self-gelled polysaccharide and wherein the second continuous phase is a portion of the matrix which defines the convective pores which are non-cylindrical large diameter continuously connected convective pores in-between the first continuous phase, wherein the non-cylindrical large diameter continuously connected convective pores have a median pore diameter of from 0.10 µm to 30 µm, and wherein the third inert phase does not constitute the structure of the second continuous phase.

The tangential flow filtration/chromatography systems of the present invention advantageously allow to simultaneously separate one or more target molecules from cells, cell debris and further contaminants contained in fermentation broth or any other particulate and impurity containing, non-clarified liquid. That is, the fermentation broth or any other non-clarified liquid is clarified from the bioreactor in a single process step using the tangential flow filtration (TFF) principle, and the one or more target molecules (often proteins) are isolated from the bioreactor broth in the same process step by chromatography.

The membrane of the above tangential flow filtration/chromatography systems of the present invention either has chromatographically active centers for selectively binding the one or more target molecules, or has chromatographically active centers for selectively binding the further contaminants.

The tangential flow filtration/chromatography systems of the present invention comprise a tangential flow filtration/chromatography device comprising at least one fluid inlet, at least one retentate outlet, at least one permeate outlet, and at least one membrane which separates a retentate section from a permeate section in the tangential flow filtration/chromatography device, the at least one retentate outlet being in the retentate section and the least one permeate outlet being in the permeate section. A flow from the at least one fluid inlet to the at least one retentate outlet is guided in the tangential flow filtration/chromatography device tangentially along the surface of the at least one membrane.

In a specific embodiment of the present invention, at least one flow fitting is arranged in the retentate section and/or in the permeate section. In order to reduce the formation of a cover layer on the filter medium and to achieve a sufficient filtration performance, suitable fabrics or nonwovens can be used as flow fittings. Examples of flow fittings can be found in EP 1 089 805 B1 or US 2014/0231339 A1.

At least one membrane is appropriately arranged within the tangential flow filtration/chromatography device to separate the retentate section and permeate section by the membrane. In this regard, a seal may be formed of e.g. a resilient material capable of being resetted, such as a silicone, a rubber, or a thermoplastic elastomer (TPE). This material can then in turn be welded, clamped or overmolded to the housing of the tangential flow filtration/chromatography device in a fluid-tight manner.

The structure of such tangential flow devices (without the specific filtration/chromatography membrane of the present invention) are known in the art e.g. from WO 2019/057652 A1. The tangential flow filtration/chromatography device used in the present invention may e.g. be a plate frame module as well as a hollow fiber module, a tube module, a spiral wound module or any other format (cf. for example DE 196 43 851 B4, US 8,747,980 B2, US 8,991,027 B2). Figure 1 depicts an exemplary tangential flow filtration/chromatography system in accordance with the present invention, in which a tangential flow filtration/chromatography device is in its center. The system comprises a feed line, a permeate lane and a retentate line. Either the elution or equilibration buffer or the fluid can flow into the tangential flow filtration/chromatography device via the feed line. Likewise, the waste can exit the tangential flow filtration/chromatography device. Either the retentate or the waste can exit the tangential flow filtration/chromatography device via the retentate line. Via the permeate line, either the permeate or the elution can leave the tangential flow filtration/chromatography device or the wash (buffer), a re-equilibration buffer and a CIP (clean-in-place) buffer can flow into the tangential flow filtration/chromatography device.

Flow distributers in the retentate section and/or permeate section can promote a more uniform incident flow of the chromatographic material. This can increase the concentration of the eluted target substance, especially during elution of the target molecule, but also purification, as well as reduce the required amounts of buffer and purification substances (cf. for example US 2021/0039015 A1 and US 6,942,794 B2).

According to the present invention, the target molecules are not subject to any particular restriction, as long as the target molecules have a maximum size (hydrodynamic radius) that allows the target molecules to permeate into and through the at least one membrane to the permeate section of the tangential flow filtration/chromatography device. The target molecules may e.g. be molecules having a hydrodynamic radius of up to 40 nm. As specific target molecules, proteins, recombinant proteins, mAbs, bispecific mAbs, enzymes, exosomes, viral vectors, viruses, nucleic acids, mRNA, DNA, etc. may be mentioned.

According to the present invention, the cells, the cell debris and the further contaminants from which the target molecules should be separated are not subject to any particular restriction, as long as the cells and the cell debris both have a specific minimum size larger than the convective pore size of the at least one membrane of the tangential flow filtration/chromatography device, whereas the further contaminants have a specific maximum size smaller than or equal to the convective pore size of the at least one membrane of the tangential flow filtration/chromatography device. That is, according to the present invention, cell debris is defined as having a specific minimum size larger than the convective pore size of the at least one membrane of the tangential flow filtration/chromatography device, whereas the further contaminants are defined as having a specific maximum size smaller than or equal to the convective pore size of the at least one membrane of the tangential flow filtration/chromatography device. The cells, cell debris and further contaminants may e.g. originate from a cultivation process of the one or more target molecules and include molecules and particles (particulate matter) stemming from a fermentation process, like cells and parts of cells, cell membrane, host cell proteins, membrane proteins, endotoxins, nucleic acids like DNA, RNA, buffer components, cell nutrients, solvents, colloids, inert particulate matter used as cell carriers or for any other purpose necessary for fermentation and superstructures thereof, etc.

The tangential flow filtration/chromatography systems of the present invention comprise a tangential flow filtration/chromatography device comprising at least one membrane which has chromatographically active centers for selectively binding either the one or more target molecules or the further contaminants.

According to the present invention, "chromatographically active centers" (which may also be called "ligands") are sites or groups attached to the at least one membrane that are capable of interacting with either the one or more target molecules or the further contaminants to be adsorbed that are present in a fluid, and are capable of selectively binding either the one or more target molecules or the further contaminants. The chromatographically active centers according to the invention are not subject to any particular restriction. According to one embodiment of the present invention, the chromatographically active centers are selected from the group consisting of ion exchangers, chelating agents and heavy metal chelates, thiophilic, hydrophobic ligands of various chain lengths and configurations, reversed-phase systems, dye ligands, affinity ligands, amino acids, coenzymes, cofactors and analogs thereof, substrates and analogs thereof, endocrine and exocrine substances, such as hormones and substances with hormone-like action, effectors and analogs thereof, enzyme substrates, enzyme inhibitors and analogs thereof, fatty acids, fatty acid derivatives, conjugated fatty acids and analogs thereof, nucleic acids, such as DNA, RNA and analogs and derivatives thereof (single-stranded, double-stranded and/or multi-stranded), and also peptide nucleic acids and derivatives thereof, viruses, virus particles, monomers and analogs and derivatives thereof, oligomers to polymers and analogs and derivatives thereof, high-molecular-weight carbohydrates, which may be linear or branched, unsubstituted or substituted, polymeric glycoconjugates, such as heparin, amylose, cellulose, chitin, chitosan and monomers and oligomers thereof and derivatives and analogs thereof, lignin and derivatives and analogs thereof, other biological/chemical ligands, such as oligopeptides and polypeptides, for example proteins and their oligomers, multimers, subunits and also parts thereof, especially lectins, antibodies, fusion proteins, haptens, enzymes and subunits and also parts thereof, structural proteins, receptors and effectors and also parts thereof, additionally xenobiotics, pharmaceuticals and active pharmaceutical ingredients, alkaloids, antibiotics, biomimetics, etc. According to one embodiment of the present invention, the chromatographically active centers are selected from the group consisting of anionic and cationic groups, hydrophobic groups, affinity ligands, metal chelates and reactive epoxide, aldehyde, azlactone, N-hydroxysuccinimide and/or carbodiimide groups. According to a particularly preferred embodiment of the present invention, the chromatographically active centers are affinity ligands such as protein A or protein B.

Introducing "chromatographically active centers" into the at least one membrane is well-known to those skilled in the art.

The at least one membrane comprised in the device of the system where the at least one membrane has chromatographically active centers for selectively binding the one or more target molecules further has a specific convective pore size to
(i) block the cells and cell debris both having a specific minimum size larger than the convective pore size from permeating into and through the at least one membrane to the permeate section, and
(ii) allow the further contaminants having a specific maximum size smaller than or equal to the convective pore size to permeate into and through the at least one membrane to the permeate section.

The at least one membrane comprised in the device of the system where the at least one membrane has chromatographically active centers for selectively binding the further contaminants further has a specific convective pore size to
(iii) block the cells and cell debris both having a specific minimum size larger than the convective pore size from permeating into and through the at least one membrane to the permeate section, and
(iv) allow the one or more target molecules to permeate into and through the at least one membrane to the permeate section.

According to the present invention, the "convective pores" are flow through pores (macropores) which provide the flow passages or channels through the membrane. According to the present invention, the convective pores have a median pore diameter of from 0.10 µm to 30 µm, preferred from 0.50 µm to 10 µm and most preferred from 1.0 µm to 5.0 µm. The median pore diameter may e.g. be determined as the median value of all pore data from the method in the scientific publication of Ley et. al, Journal of Membrane Science, Volume 564, 2018, Pages 543-551 (Page 546-548).

According to the present invention, the at least one membrane having chromatographically active centers comprises a polymer network material-based self-supporting bi-continuous separation matrix comprising a first continuous phase and a second continuous phase, the at least one membrane optionally comprising a third inert phase, wherein the first continuous phase is a portion of the matrix which is formed by a self-gelled polysaccharide and wherein the second continuous phase is a portion of the matrix which defines non-cylindrical large diameter continuously connected convective pores in-between the first continuous phase, wherein the large diameter continuously connected convective pores have a median pore diameter of from 0.10 µm to 30 µm, and wherein the third inert phase does not constitute the structure of the second continuous phase. Such membranes are described in detail in international application PCT/EP2021/087131.The self-gelled polysaccharide phase is modified by introducing the chromatographically active centers, which is well-known to those skilled in the art.

In particular, the at least one membrane used in the present invention comprises a separation matrix which is a self-supporting separation matrix. As used herein, the expression "self-supporting separation matrix" means that the separation matrix, due to its continuous connected gel structure, is inherently dimensionally stable, and can be generated and handled without the need of an inert substrate as a supporting substrate. This does not exclude the possibility to introduce a third inert phase as a reinforcing substrate if needed for further enhanced mechanical strength or elevated ease of handling or integration into a chromatographic device.

The self-supporting matrix of the at least one membrane used in the present invention has a bi-continuous structure. As used herein, the expressions "bi-continuous" or "bicontinuous" mean that the separation matrix has a structure which comprises two voluminous (mostly) continuous phases that penetrate each other and are each (mostly) continuous (interconnected) in themselves. Thus, at least 90%, preferably at least 95%, and more preferably at least 98% of the volume elements within a (mostly) continuous phase can be reached from any point within this voluminous phase without leaving this phase. According to a particular preferred embodiment, every volume element within a continuous phase can be reached from any point within this voluminous phase without leaving this phase. This can be seen in 3D structures of the separation matrix by inspecting a volume element visually of at least 64*10³ µm³. How to obtain 3D structures by Confocal Laser Scanning Microscopy is known to a person skilled in the art of the present technical field and an example thereof can be found in the scientific publication of Ley et. al, Journal of Membrane Science, Volume 564, 2018, Pages 543-551 (Page 545).

The first continuous phase of the separation matrix of the at least one membrane used in the present invention is a portion of the matrix which is formed by a self-gelled polysaccharide, i.e. the first continuous phase comprises at least 90% of a self-gelled polysaccharide, preferably at least 95%, more preferably at least 98%, and more preferably at least 99%. According to a particularly preferred embodiment, the first continuous phase comprises 100% of a self-gelled polysaccharide, i.e. the first continuous phase consists of a self-gelled polysaccharide. The first continuous phase typically has small diameter diffusion pores in a molecular dimension of about 10 to 500 nm. The first phase is (mostly) continuously connected, i.e. at least 90%, preferably at least 95%, and more preferably at least 98% of the volume elements within the first phase can be reached from any point within the first phase without leaving the first phase. According to a particular preferred embodiment of the present invention, every volume element within the first phase can be reached from any point within the first phase without leaving the first phase.

As used herein, "gelling" is the process in which a polymer solution, particularly a polysaccharide solution, solidifies and therefore forms a polymer gel in which a solvent (such as e.g. water or glycerin) is filling the porous space inside the polymer gel. The solvent may improve the (storage) stability of the gel. For a self-gelling/self-gelled polymer/polysaccharide gel, no external or additional polymer or monomer or cross linker has to be involved to form the physical or covalent bonds that are leading to the polymer/polysaccharide gel formation.

Polysaccharide gels play an important role for the manufacture of materials for separation of mixtures of biomolecules. Among the characteristics making these gels especially interesting can be mentioned their inertness (as long as no chromatographically active centers have been introduced into the polysaccharide gel) in contact with proteins and other biomolecules and their porous structure. A further important property is their resistance against alkaline conditions, which is of great importance in large scale separation processes requiring frequent regeneration/sterilization of the gel.

Generally, polysaccharide gels are used for various types of chromatography. One such example is gel filtration, whereby the sample constituents are size fractionated. The choice of polysaccharide gels as the carrier matrix is based on their inertness and a well-established derivatization chemistry for the introduction of ion exchange groups and a number of convenient techniques available for introducing e.g. affinity ligands and due to the minimum of unspecific binding of biomolecules.

Polysaccharide gels typically have small diameter diffusion pores in a molecular dimension of about 10 to 500 nm, which may only be reached by components (target substances and/or contaminants) in the mobile phase via diffusion. In order to effectively transport binding components to the gel, a convective phase surrounds the polysaccharide gel, forming an interface between the convective phase and the diffusive gel phase. The convective phase is formed by large diameter convective pores (also called "flow through pores" or "macropores") which provide flow passages or channels, through the gel. The solidified polysaccharide gel forms so-called "bridges" around the large diameter convective pores. The small diameter diffusion pores are within said bridges formed by the solidified polysaccharide. When such porous polysaccharide gels are packed in a chromatographic column and a liquid flow is applied through the column, the flow will mainly pass through the gel via the large diameter convective pores. The components (target molecules and/or contaminants) to be separated are thus transported also to the inner parts of the gel by convective flow, which is a much faster way of transportation than diffusion. Therefore, only short distances have to be covered by diffusion in such porous polysaccharide gels. This high reachability allows target molecules or contaminants to diffuse quickly into the gel to bind. On the other hand, the target molecules or contaminants can also diffusively leave the gel quickly after changing the buffer conditions (pH, ionic strength, etc.), and can thus be convectively eluted from the membrane. This high diffusive accessibility allows high mass transport to the binding sites of the membrane, thus enabling high flow rates or productivity without breakthrough/loss of the target molecule. At the same time, the short diffusive distance allows effective elution of the target molecule.

The smaller the diameter of the pores is, the smaller also the "bridges" / diffusive path length (which is half the length of the bridge diameter) is. The correlation between large diameter convective pores and bridges is accessible through geometrical considerations. In particular, the bigger the large diameter convective pores become with a constant pore volume fraction (convective porosity), the bigger the bridges have to become because the bridges just represent the space between the large diameter convective pores. If there are less large diameter convective pores (bigger pores with constant volume fraction also means less pores) the space between them has to grow. Therefore, in case a porous polysaccharide material has rather small large diameter convective pores, the diffusive path length within the bridges is also rather small and the diffusive pores within the porous bridges are accessible within very short diffusion times. This geometrically given correlation indicates strongly why a smaller pore size is advantageous for a chromatographic operation within such materials.

The self-gelled polysaccharide (having small diameter diffusion pores) is porous, i.e. the polysaccharide has at least 20% porosity. The pores within the self-gelled polysaccharide are small diameter diffusion pores typical to polysaccharide gels. Porosity as used herein is defined as the volume of the material filled by a gas or a liquid (*V_{gas or liquid or both in parts}*) divided by the whole volume of the material including the solid volume (*V*_{*total gas*+*liquid*+*solid*}). $porosity = \frac{{V}_{gas or liquid or both in parts}}{{V}_{total gas + liquid + solid}}$

According to a preferred embodiment of the present invention, the self-gelling/self-gelled polysaccharide is one or more selected from the group consisting of agarose, agar, agaropectin, kappa-carrageenan, iota-carrageenan, lambda-carrageenan, gellan gum, amylose, curdlan, alginate and rhamsan gum. These self-gelling/self-gelled polysaccharides advantageously allow to form a separation matrix having small pores and large pores, i.e. a structure having small diameter diffusion pores and large diameter convective pores (double porous structure), by using a single emulsion during preparation. Moreover, said hydrophilic polysaccharides exhibit low non-specific binding. According to a particularly preferred embodiment of the present invention, the self-gelling/self-gelled porous polysaccharide is agarose. Functionalized agarose is known to have a very high specific binding for biological molecules and at the same time a very low non-specific binding. Thus, functionalized agarose can very effectively separate target molecules from the further contaminants.

The second continuous phase of the separation matrix of the at least one membrane used in the present invention is a portion of the matrix which defines non-cylindrical large diameter continuously connected convective pores in-between the first continuous phase. The non-cylindrical large diameter convective pores are (mostly) continuously connected (interconnected), i.e. at least 90%, preferably at least 95%, and more preferably at least 98% of the pores of the non-cylindrical large diameter convective pores can be reached from any point of another pore of the non-cylindrical large diameter convective pores by moving only through non-cylindrical large diameter convective pores. According to a particularly preferred embodiment of the present invention, any pore of the non-cylindrical large diameter convective pores can be reached from any point of another pore of the non-cylindrical large diameter convective pores by moving only through non-cylindrical large diameter convective pores. The non-cylindrical large diameter convective pores are in-between the first continuous phase (the self-gelled polysaccharide), i.e. the self-gelled polysaccharide forms porous gel bridges around the non-cylindrical large diameter convective pores. Said large diameter convective pores are non-cylindrical, i.e. said pores are substantially spherical wherein parts of the substantially spherical pores typically overlap in order to form a continuous convective pore structure.

According to the present invention, the non-cylindrical large diameter continuously connected convective pores have a median pore diameter of from 0.10 µm to 30 µm. Before international application PCT/EP2021/087131, no membrane comprising a polymer network material-based self-supporting bi-continuous separation matrix without an inert phase as supporting material for constituting the second continuous (pore) phase of the separation matrix has been described in the art that has such small non-cylindrical continuously connected large diameter convective pores, since no suitable method for preparing such membrane was known. The median pore diameter may e.g. be determined as the median value of all pore data from the method in the scientific publication of Ley et. al, Journal of Membrane Science, Volume 564, 2018, Pages 543-551 (Page 546-548). Preferably, the median pore diameter is from 0.50 µm to 10 µm and most preferred from 1.0 µm to 5.0 µm. Since the non-cylindrical continuously connected large diameter convective pores have a median pore diameter within the above range, the diffusive path length is also small and the small diameter diffusive pores within the porous bridges are accessible within very short diffusion times, advantageously allowing to operate chromatographic processes very quickly and efficiently.

The proportion of the polysaccharide phase is independent of the convective pore size. Therefore, the binding capacity is also independent of the convective pore size. Therefore, the convective pore size can be adjusted according to other criteria, e.g., to achieve ideal depletion of cells or cell debris in tangential flow, or to minimize membrane fouling during the loading phase, or to achieve high membrane permeability. High permeability of the membrane can achieve higher flow through the convective phase of the membrane at the same transmembrane pressure, or thicker membrane beds can be constructed while maintaining sufficiently high flux conductivity.

A thicker membrane bed results in a lower aspect ratio for the same bed volume (=binding capacity), which in turn results in improved upstream and downstream flow of the elution buffer, or lower dispersion. The lower dispersion means that the entire membrane can be eluted with less elution buffer (buffer savings compared to less thick beds). Using a lower amount of buffer while completely eluting all target molecules increases the concentration of the target molecule in the elution pool. A high concentration in the elution pool is highly desirable, as a further step for concentration can possibly be omitted or smaller elution vessels are required.

Thicker beds and thus lower aspect ratios can also minimize the dead volume in the device, which leads to lower dispersion, especially when eluting the target molecules.

Although the separation matrix of the at least one membrane used in the present invention is a self-supporting inherently stable separation matrix, the at least one membrane used in the present invention may also optionally comprise a third inert phase as a reinforcing material to even more strengthen the chromatographic material against external stresses such as pressure or damaging radiation. In case a third inert phase is present as a reinforcing material, the third inert phase does not constitute the structure of the second continuous phase of the separation matrix. That is, the third inert phase is randomly distributed in the polymer network material-based self-supporting bi-continuous separation matrix independently from the structure of the second continuous phase, and the pore structure of the second continuous phase does not follow the structure of the inert phase like in the chromatographic materials described in US 7,479,233 B2.

The third inert phase as an additional reinforcing material for the separation matrix in the at least one membrane used in the present invention may facilitate production and use of the membrane due to increasing the stability of the separation matrix. The third inert phase acting as a reinforcing material is not particularly limited and may e.g. be fleece materials or nonwovens (drylaid, wetlaid, spunlaid, meltblown), wovens, fabrics, mesh fabrics, open cell foams, reticulated foams or other permeable porous materials. Commercially available reinforcing materials are e.g. nonwoven Novatexx 2465 from Freudenberg; nonwoven Reemay 2016 from Berry, and mesh fabric Sefar 07-200/35.

According to a preferred embodiment of the present invention, the at least one membrane used in the present invention additionally comprising a third inert phase has a ratio of the volume of the third inert phase to the total volume of the membrane including the third inert phase of from 0.03 to 0.60, more preferred of from 0.05 to 0.40, most preferred of from 0.08 to 0.25. As used herein, the "volume of the third inert phase" (V) is the volume that is taken by the third inert phase. By knowing the density (*φ*) of the underlying material, the volume of the third inert phase (V) can be calculated from the mass (m) thereof:${V}_{third inert phase} = \frac{{m}_{third inert phase}}{{φ}_{third inert phase}}$

As used herein, the "total volume of the membrane including the third inert phase" is the total volume of the physical dimensions of the membrane including the third inert phase volume and the volume of the two continuous phases. Said total volume can be determined by measuring the outer dimensions like diameter and thickness of the membrane, depending on its macroscopic appearance.

If the aforementioned volume ratio is below the above range, the stability enhancing effect of the third inert phase acting as reinforcing material may be diminished. If the aforementioned volume ratio is above the aforementioned range, the binding capacity and the permeability of the membrane will be diminished.

The third inert phase acting as a reinforcing material preferably has one or more of the following properties:
- base stability, i.e. the reinforcing material resists 0.1N NaOH for at least 5 h, more preferably for at least 24 h, without significant degradation or loss of functionality;
- gamma stability, i.e. the reinforcing material resists at least 10 kGy, more preferably 25 kGy, most preferably 50 kGy, without significant degradation or loss of functionality;
- a tensile strength: > 5.2 MPa, preferred > 6,8 MPa (Tensile tests are performed using a 2.5 kN testing machine (ZWICK & ROLL). The temperature during measurements is 20 °C/room temperature. Tensile specimen are 150 mm long, 20 mm wide. Each specimen is placed centrally in the testing machine's specimen mounts. Pulling speed for all measurements is 50 mm/min. The value tensile strength is defined as the highest tensile strength at sample break (Rm).);
- compressibility: ≥ 85%, preferably > 90% of the initial thickness remains. In order to measure the compressibility, the following method is used. Hereby the thickness is measured while a defined pressure is on the material, and over time the reduction of thickness is measured. Thickness measurements were carried out with an Universal Micrometer (FRANK PTI GmbH). A round push rod with an area of 10 cm² is used as specimen mount. Round samples of 47 mm diameter are placed between the plate and the push rod. For a compressibility measurement thickness is measured by the compression of a weight of 1 kg that is placed on top of the push rod while monitoring the sample's thickness for 5 min. Compression is calculated by: $Compression \left[%\right] = \frac{thickness after compression with 1 Kg}{thickness before compression with 1 Kg} ⋅ 100$
- a homogeneity in thickness and density of at most 10% variation around the mean value.

Due to the presence of an additional reinforcing material, the processability (rinsing, impregnation, drying of roll material) in the x/y direction of the separation matrix can be advantageously increased since the force absorption without significant length change (elongation) in the pull direction for possible production/modification processes (weaving processes) can be increased when compared to an embodiment without reinforcing material. In addition, the choice of material of the reinforcing material (e.g. a thermoplastic resin) can ensure an improved attachment of the separation matrix to thermoplastic device parts of a chromatography unit. Accordingly, the manufacturability of an integrated chromatography device can be improved by an additional reinforcing material.

According to a preferred embodiment, the volume ratio of the self-gelled polysaccharide to the total volume of the at least one membrane including the third inert phase is at least 30 vol%, more preferably at least 40 vol% and most preferably at least 50 vol%.

According to a preferred embodiment of the present invention, the median bridge diameter of the first continuous phase is from 0.4 to 4 times larger than the median pore diameter of the non-cylindrical large diameter continuously connected convective pores. This ratio is strongly dependent on the convective porosity ε (e.g. in case ε = 0.33 (i.e. at a convective porosity of 33%) so that 4.4· *e*^{-0.03·(*ε*·100)} is equivalent to a bridge to pore ratio of 1.63), as described by the following equation. $bridge to pore ratio = \frac{median bridge diameter \left[μm\right]}{median pore diameter \left[μm\right]} = 4.4 ⋅ {e}^{- 0.03 ⋅ \left(ε⋅100\right)}$

As used herein, the median bridge diameter is defined as the median value of all pore data from the method in scientific publication Ley et. al, Journal of Membrane Science, Volume 564, 2018, Pages 543-551, when the phases for the binary structure where inverted (1 goes 0 and vice versa) (cf. Pages 546-548 thereof). Preferably, the median bridge diameter of the first continuous phase is from 1 to 3 times larger than the median pore diameter of the non-cylindrical large diameter continuously connected convective pores, more preferably from 1 to 2 times larger. A ratio outside of the above range may negatively affect the surface accessibility, and thus also the binding or process time.

According to a preferred embodiment, when a convective porosity ε is the ratio of the volume of the second continuous phase to the total volume of the first and second continuous phase, the convective porosity ε is from 0.05 to 0.7, more preferred from 0.05 to 0.5, and most preferred from 0.1 to 0.4. The convective porosity ε can be determined by inverse size exclusion chromatography as known by a person skilled in the art and shown exemplary in a modified form in Guan, Hong & Guiochon, Georges (1996) "Study of physico-chemical properties of some packing materials" (Journal of Chromatography A - J CHROMATOGR A. 731. 27-40. 10.1016/0021-9673(95)01197-8). As used herein, the "total volume of the first and second continuous phase" refers to the sum of the volume of the first phase and the volume of the second phase, without taking an optional inert third phase (and its volume) in the at least one membrane preferably used in the present invention into account. If the convective porosity is smaller than the above-defined range, the permeability of the membrane may decrease due to the presence of a larger amount of polysaccharide material in the membrane. If the convective porosity is larger than the above-defined range, the binding capacity of the membrane may decrease due to the presence of a smaller amount of polysaccharide material in the membrane.

According to a preferred embodiment of the present invention, the at least one membrane used in the present invention has a permeability of at least 0.31×(ε×100) mD (milliDarcy), more preferred at least 1.52×(ε×100) mD and most preferred at least 3.1×(ε×100) mD, wherein ε is the convective porosity as defined above. Accordingly, when the convective porosity ε is 0.33 (i.e. a convective porosity of 33%), the membrane preferably has a permeability of at least 10.2 mD, more preferably at least 50.2 mD, and most preferably at least 102 mD. The permeability strongly depends on the convective porosity ε. This is accessible through geometrical considerations. At constant median pore diameter and a higher convective porosity, a higher number of pores has to be present and therefore a higher volume flow at constant pressure. For smaller convective porosities vice versa. The permeability may e.g. be determined as described in scientific publication Ley et. al, Journal of Membrane Science, Volume 564, 2018, Pages 543-551 (page 547: description; page 545: measurement). The higher the permeability is, the smaller the required process pressure in chromatographic processes using the embrane is. This advantageously allows greater column lengths and greater bed heights in chromatographic processes and therefore lower axial dispersion. Moreover, determination of the permeability is also a method to confirm a bi-continuous structure of the separation matrix since a permeability of lower than 0.31×(ε×100) mD (e.g. 10.2 mD at a convective porosity of 33% (i.e. ε = 0.33)) is a strong indication of the absence of a continuous convective phase.

According to a preferred embodiment of the present invention, the coefficient of variance of the median pore diameter of the non-cylindrical large diameter continuously connected convective pores is at most 0.8, more preferred at most 0.7. As used herein, the coefficient of variance of the median pore diameter is the standard deviation of the median pore diameter divided by the mean value of the median pore diameter. The "standard deviation" of the median pore diameter may be determined by using the Microsoft Excel function "STABW.N" which calculates the standard deviation based on a population specified as arguments (logical values and text are ignored). The standard deviation is a measure of the dispersion of values with respect to their mean (the mean). If the coefficient of variance of the median pore diameter of the non-cylindrical large convective pores is higher than the above maximum, the performance of the membrane in a chromatographic process may decrease, i.e. earlier breakthrough curves, broader elution peaks and smaller chromatographic resolution may occur.

According to a preferred embodiment of the present invention, the coefficient of variance of the median bridge diameter of the first continuous phase is at most 0.7, more preferred at most 0.6. As used herein, the coefficient of variance of the median bridge diameter is the standard deviation of the median bridge diameter divided by the mean value of the median bridge diameter. The standard deviation of the median bridge diameter is determined analogously to the method described above for the standard deviation of the median pore diameter. If the coefficient of variance of the median bridge diameter of the first continuous phase is higher than the above maximum, it may occur that the binding sites of the separation matrix cannot be fully utilized, leading to earlier breakthrough and broader elution peaks in chromatographic processes.

According to a further preferred embodiment of the present invention, the at least one membrane used in the present invention has a specific interface between the first and the second continuous phases of at least 0.04 m²/mL (wherein the volume mL refers to the separation matrix volume without the optional third inert phase/reinforcing material), more preferred more than 0.1 m²/mL and most preferred more than 0.2 m²/mL. The specific interface between the first and the second continuous phases can be determined by the following equation if the tortuosity is 2.5. If the tortuosity is different from this exemplary but typical tortuosity than the permeability changes by a factor that the then observed tortuosity differs from 2.5 to higher permeabilities (for lower tortuosities) or lower permeabilities (for higher tortuosities) like it is known to a person skilled in the art and given in the following equation (cf. also the scientific publication Berg et. al, Transport in Porous Media, 2014, Page 10, https://arxiv.org/pdf/1505.02424.pdf). ${Interface}_{specific} = \left(-37.868 {ϵ}^{5}+75.428 {ϵ}^{4}-66.017 {ϵ}^{3}+34.08 {ϵ}^{2}-2.7346 ϵ+0.1333\right) ⋅ {Permeability}^{- 0.5}$ ${Permeability}_{with tortuosity different than 2.5} = \frac{{Permeability}_{with a tortuosity of 2.5}}{\frac{tortuosity}{2.5}}$

If the specific interface between the first and the second continuous phases is below 0.04 m²/mL, the interface for the transition into the small diameter diffusion pores may be too small such that less components (target substances and/or contaminants) may diffuse into the first continuous phase. Accordingly, the mass transport and thus the binding capacity may be limited while the diffusion time remains the same, leading to either smaller binding capacity or longer process times.

The method of producing the at least one membrane used in the present invention is described in detail in international application PCT/EP2021/087131.

In particular, a method of producing the membrane comprising a polymer network material-based self-supporting bi-continuous separation matrix, wherein the first continuous phase is formed by a self-gelled polysaccharide, used in the present invention is a method comprising the steps of:
(a) preparing a solution (A) comprising a self-gelling polysaccharide and a first solvent;
(b) preparing a solution (B) comprising at least one surfactant and a second solvent which is an immiscible solvent to solution (A);
(c) combining solution (A) and solution (B) to produce a combined solution (C);
(d) emulsifying the combined solution (C) at a condition to allow the self-gelling polysaccharide to remain in solution in the first solvent as solution (A) to obtain an emulsion which is in form of a solution (B)-in-solution (A)-emulsion; and
(e) allowing solution (A) containing the self-gelling polysaccharide to solidify by gelation at a certain condition to form the membrane which comprises the separation matrix comprising the self-gelled polysaccharide as the first continuous phase.

In step (a) of the method, a solution (A) comprising a self-gelling polysaccharide and a first solvent is prepared. The first solvent is not particularly limited and every solvent in which the self-gelling polysaccharide is soluble (at room temperature or at elevated temperature) can be used. The first solvent may also be a mixture of two or more of such solvents. According to a preferred embodiment, the first solvent is water. Solution (A) may be prepared by stirring and heating the self-gelling polysaccharide and the first solvent (to e.g. 30 °C or more, 40 °C or more, 50 °C or more, 60 °C or more, 70 °C or more, 80 °C or more, or 90 °C or more) for a predetermined time (e.g. 2 min or more, 5 min or more, 10 min or more, 15 min or more) until the self-gelling polysaccharide is (completely) dissolved in the first solvent.

The concentration of the self-gelling polysaccharide is not particularly limited. According to a preferred embodiment, the concentration of the polysaccharide in solution (A) is from 0.5 to 8 wt%, preferably from 1 to 6 wt%, and most preferably from 2.5 to 5 wt%. If the concentration is below the above range, the gelling ability of the self-gelling polysaccharide may not be sufficient, which may lead to deterioration of the stability of the polysaccharide gel. If the concentration is above the aforementioned range, unwanted size exclusion of a target component to be bound in a chromatography process and/or exceedingly low diffusive mass transfer into the gel may occur.

In step (b) of the method, a solution (B) comprising at least one surfactant and a second solvent which is an immiscible solvent to solution (A) is prepared. As used herein, "immiscible" means that the solubility of a solvent in another solvent is less than 100 g/L at 70 °C, preferably less than 50 g/L at 70 °C, and most preferably less than 10 g/L at 70 °C.

The second solvent may be a single solvent or a mixture of two or more solvents.

At least one solvent of the second solvent is an immiscible solvent to solution (A).

According to a preferred embodiment, the second solvent is one or more selected from C₄₋₁₂ alcohols (e.g. 1-hexanol, 2-heptanol, 1-octanol, 1-nonanol, 1-decanol, 1-dodecanol), C₄₋₁₂ isoalcohols, alkanes (e.g. octane and decane), silicone oils having a viscosity of from 4×10⁻³ Pa·s (4 cP) to 2×10 Pa·s (200 cP) and natural oils having a viscosity of from 4×10-3 Pa·s (4 cP) to 2×10⁻¹ Pa·s (200 cP) or mixtures thereof. Silicone oils may e.g. be polydimethylsiloxanes (e.g. Silicone oil 20 cP from Wacker). Natural oils may e.g. be olive oil, sunflower oil or rapeseed oil. Particularly preferred embodiments of the second solvent are octane, decane, 1-octanol, 1-decanol and 1-dodecanol. The most preferred second solvent is decanol, particularly 1-decanol. Solution (B) may be e.g. prepared by stirring at room temperature or at elevated temperature, such as e.g. 30 °C or more, 35 °C or more, 40 °C or more, or 50 °C or more, for a predetermined time (e.g. 2 min or more, 5 min or more, 10 min or more, 15 min or more).

According to a preferred embodiment, the at least one surfactant has an HLB value of from 8 to 16, preferably of from 10 to 14, and most preferably of from 11 to 13. The HLB (hydrophilic-lipophilic balance) of a surfactant is a measure of the degree to which it is hydrophilic or lipophilic, determined by calculating values for the different regions of the molecule, as described by Griffin ("Classification of Surface-Active Agents by 'HLB"', Journal of the Society of Cosmetic Chemists, 1949, 1 (5): 311-26); and "Calculation of HLB Values of Non-Ionic Surfactants", Journal of the Society of Cosmetic Chemists, 1954, 5 (4): 249-56) and Davies ("A quantitative kinetic theory of emulsion type, I. Physical chemistry of the emulsifying agent", Gas/Liquid and Liquid/Liquid Interface, Proceedings of the International Congress of Surface Activity, 1957, pp. 426-38). Taking tabulated or calculated values for the HLB values of single surfactants, HLB values of mixtures are determined by the mass fraction like e.g. in the following formula for a mixture of 2 surfactants where *m* is the mass of the surfactant used and HLB is the tabulated or calculated HLB value for the specific surfactant. ${HLB}_{mixture \left(of 2 surfactants\right)} = \frac{{m}_{1} ⋅ {HLB}_{1} + {m}_{2} ⋅ {HLB}_{2}}{\left({m}_{1}+{m}_{2}\right)}$

The smaller the HLB-value is, the smaller the resulting convective pore diameter and resulting bridge diameter are. The HLB value is an independent parameter with respect to the volume of the polysaccharide phase (first continuous phase) and the polysaccharide concentration. If the volume ratio of the two continuous phases (first continuous phase and second continuous phase) is used to control the pore size, it also changes the convective porosity and consequently also the permeability. If the polysaccharide concentration is used to control the pore size of the second continuous phase (pore phase), the first continuous phase (polysaccharide phase / diffusive phase) is also changed. For example, when using 1-decanol as organic solvent, at higher polysaccharide concentrations, the convective pores become larger and, at the same time, the diffusive pores become smaller which may lead to exclusion of larger molecules and reduced diffusive mass transport. Conversely, again using 1-decanol as organic solvent, at lower polysaccharide concentrations, the convective pores become smaller and the diffusive pores become larger, which improves mass transport but also reduces the surface area for possible binding of target components to the separation matrix in a chromatography process. By using the above HLB-value range, it is advantageously possible to influence the convective pore size without these effects.

In view of the above, the HLB-value of the at least one surfactant is preferably within the above HLB-value range, and the at least one surfactant is further capable of generating a bi-continuous structure. Moreover, the use of at least one surfactant preferably yields an interfacial tension between both liquid phases in an emulsion of at most 1 mN/m, and is preferably non-ionic. Surface tensions between two phases were measured with a goniometer OCA 15Pro and the pendant drop method. The needle of a syringe filled with a liquid phase A, exemplary water, but all other solutions and mixtures are possible, was placed into a liquid phase B, exemplary any organic solvent that is immiscible with water, but all other solutions and mixture of liquids are possible. A droplet of phase A was dispensed in phase B, either by volume, e.g. 10 µL, or continuously with a dispense rate of e.g. 1 µL/s, and recorded with a camera placed 90° to the needle. From the curvature of the droplets and the diameter of the needle, surface tensions between phases A and B were calculated with the software SCA 22 - surface/interfacial tension from Dataphysics Instruments (cf. F. K. Hansen, G. Rodsrud, Surface Tension by Pendant Drop, Journal of Colloid and Interface Science, Vol. 141, No. 1, 1991).

Preferred surfactants are polysorbates and/or C**₂₋₁₅₀** fatty acid esters of sorbitol, such as e.g. commercially available Tween 20/60/80 (Tween 20: polyoxyethylene sorbitan mono-laurate; Tween 80: polyoxyethylene sorbitan mono-oleate) or Span 20/60/80/85. One single surfactant or a combination of two or more surfactants may be used. According to a particularly preferred embodiment, two different surfactants are used in combination.

For manufacturing a bi-continuous structure (and not a foam structure), a minimum surfactant concentration has to be used depending on the used organic solvent and on the polysaccharide concentration. For example, based on the volumetric ratio of the total volume of the solvents (i.e. both of the first and second solvent) and the surfactant(s), the preferred amount of surfactant is calculated by the formula given below and describes only the percentage of surfactant that is at least present in solution (B), and the preferred amount is at least 2.5 vol% for 1-dodecanol, at least 4.5 vol% for 1-decanol, at least 7.5 vol% for 1-nonanol, at least 7.5 vol% for 1-octanol, at least 15 vol% for 1-hexanol, at least 7.5 vol% for 2-heptanol, at least 2 vol% for cyclohexane, at least 3 vol% for octane, at least 2.5 vol% for decane, and at least 7 vol% for silicone oil (PDMS). This surfactant concentration is hereby calculated in dependence on the total amount of organic phase used by be following equation. The higher the total amount of organic phase the higher the total amount of surfactant needed. $surfactant concentration \left[in vol%\right] = \left(\frac{{v}_{%} surfactant}{{v}_{%} organic solvent}\right) \times 100$

The above surfactant concentrations (depending on the organic solvent) are particularly preferably used in case the concentration of the polysaccharide in solution (A) is from 2.5 to 5 wt%.

In step (c) of the method, solution (A) and solution (B) are combined to produce a combined solution (C). Step (c) may be carried out at room temperature or at elevated temperature, such as e.g. 30 °C or more, 35 °C or more, 40 °C or more, or 50 °C or more. According to a preferred embodiment, the volume ratio of the second solvent in the combined solution (C) in step (c) is from 10 to 70 vol% since in this range a suitable emulsion can be generated in step (d).

In step (d) of the method, the combined solution (C) is emulsified at a condition to allow the self-gelling polysaccharide to remain in solution in the first solvent as solution (A) to obtain an emulsion which is in form of a solution (B)-in-solution (A)-emulsion. According to a preferred embodiment, step (d) is carried out by exposing the combined solution (C) to a shear force by (vigorously) stirring, dispersing or homogenising, e.g. with a Turrax Specification: T25 digital ULTRA-TURRAX^{®}, Model T25D, Firma IKA (at 10.000 to 20.000 rpm) or EUROSTAR 40 digital from IKA with a dispersing disk of type R1303 Dissolver Stirrer (at 1.000 rpm to 2.000 rpm). Vigorously stirring, dispersing or homogenising advantageously enables to generate non-cylindrical large diameter continuously connected convective pores having a median pore diameter of from 0.1 µm to 6 µm.

In step (e) of the method, solution (A) containing the self-gelling polysaccharide is allowed to solidify by gelation at a certain condition to form the membrane which comprises the separation matrix comprising the self-gelled polysaccharide as the first continuous phase. Allowing the self-gelling polysaccharide to solidify by gelation at a certain condition, is e.g. realized by cooling the solution (B)-in-solution (A)-emulsion to a temperature below the gelling temperature of the polysaccharide, e.g. cooling to at most 45 °C, at most 40 °C, at most 35 °C, at most 30 °C or at most 25°C. In other embodiments, the solidification is realized e.g. by addition of e.g. potassium salts to the solution (B)-in-solution (A)-emulsion. According to a preferred embodiment, step (e) is performed by pouring the emulsion on a casting form and cooling the emulsion below the gelling point. As used herein, a "casting form" is any surface onto which the emulsion can be given/poured to solidify, and preferably this surface is a metal surface that can be cooled down quickly, and most preferably this surface is heatable and coolable in a range of from 0 °C to 100 °C. The cooling rate is not particularly limited, and usually a cooling rate of from 0.01 °C/sec to 50 °C/sec is used. According to a preferred embodiment, the cooling rate is from 0.2 °C/sec to 30 °C/sec, particularly preferred from 1 °C/sec to 20 °C/sec.

In case the membrane used in the present invention is intended to also comprise a third inert phase acting as a reinforcing material, the third inert phase is preferably present in step (e) of the above-described method to introduce the third inert phase (randomly) into the separation matrix. That is, it is preferred that the solution (B)-in-solution (A)-emulsion of step (d) is poured onto a reinforcing material (third inert phase) in step (e) and the solution (A) is allowed to solidify by gelation in the presence of the third inert phase.

According to a particularly preferred embodiment of the method, the self-gelling polysaccharide is agarose, and the method comprises the steps of:
(a) preparing the solution (A) comprising the agarose and water by heating an agarose-in-water-suspension to at least 90°C for at least 15 minutes to solve the agarose in the water;
(b) preparing the solution (B) comprising the at least one surfactant and the second solvent which is a water-immiscible organic solvent and heating solution (B) to a temperature above the gelation temperature of the used agarose (e.g. at least 40°C);
(c) combining solution (A) and solution (B) at a temperature above the gelation temperature of the used agarose (e.g. at least 40°C) to produce a combined solution (C);
(d) emulsifying combined solution (C) at a temperature above the gelation temperature of the used agarose (e.g. at least 40°C) to allow the agarose to remain in solution in the first solvent as solution (A) to obtain an emulsion which is in form of a solution (B)-in-solution (A)-emulsion; and
(e) allowing solution (A) containing the self-gelling agarose to solidify by gelation at a temperature below the gelation temperature of the used agarose (e.g. below 35°C) to form the membrane which comprises the separation matrix comprising the self-gelled agarose as the first continuous phase.

The separation matrix is modified by introducing the chromatographically active centers (functional groups and/or ligands) described hereinabove. Such modifications by introducing functional groups and/or ligands is well-known to those skilled in the art.

The membrane comprising a polymer network material-based self-supporting bi-continuous separation matrix has both diffusion pores and convective pores, the convective pores having a very small size when compared to separation matrices known in the prior art. Due to the small convective pores, the diffusive path length is also rather small and the diffusive pores within the porous bridges of the separation matrix are accessible within very short diffusion times. Moreover, the membrane shows, compared to purely convective matrices, a high permeability due to its specific structure, thus leading to a smaller process pressure when using the membrane in chromatographic processes. This advantageously allows longer column lengths in chromatographic processes und lower axial dispersion. Moreover, the membrane advantageously shows a lower fouling propensity when compared to state-of-the-art materials. In view of the above, the membrane used in the present invention advantageously allows to operate chromatographic processes very quickly and efficiently by excellent accessibility of the binding sites, late breakthrough curves and narrow elution peaks, and thus the separation matrix is particularly suited for diffusion limited processes that need high binding capacities, as e.g. observed in bind and elute applications of large proteins, virus particles and the like.

Detailed experimental data regarding the above-described membrane used in the present invention is disclosed in international application PCT/EP2021/087131.

The tangential flow filtration/chromatography device used in the present invention may further comprise a microfiltration membrane or ultrafiltration membrane in the retentate section and/or the permeate section. In a particularly preferred embodiment, the at least one membrane is protected in the retentate section by an upstream (conventional) microfiltration membrane or ultrafiltration membrane. This can reduce the penetration of particularly large particulates into the at least one membrane and reduce fouling or blocking of said membrane. In another particularly preferred embodiment, the at least one membrane can be protected in the permeate section by a microfiltration membrane or ultrafiltration membrane suitable for sterile filtration. This prevents bacteria from entering the chromatographic bed and the retentate section or enables the permeate to be discharged sterilely. The microfiltration membrane or ultrafiltration membrane used in the retentate section or in the permeate section typically has a preferred mean pore size diameter of from 0.10 to 10 µm, more preferred from 0.45 to 5.0 µm and most preferred from 0.80 to 3.0 µm.

According to a preferred embodiment of the present invention, the tangential flow filtration/chromatography device used in the present invention comprises two or more layers of membranes having chromatographically active centers, said two or more layers constituting a membrane bed. The membrane bed preferably has a thickness of from 0.10 to 40 mm, more preferably from 0.50 to 30 mm, and most preferably from 2.0 to 20 mm.

A membrane layer typically has a thickness of from 100 to 1,000 µm. Under certain circumstances, it may be useful to use one or more inert layers (interlayers) between the membrane layers, which provide mechanical and local separation of the individual membrane layers. These interlayers can, for example, be made of laid or extruded nonwovens of different materials (e.g. polymers, thermoplastics), or of other materials that improve the performance of the stack in terms of overall permeability and/or processability/integrability in the device. These interlayers are inert and are not functionalized like the membranes having chromatographically active centers, i.e. said interlayers do not exhibit specific binding for the target molecules or the further contaminants. Such interlayers are known to those skilled in the art (cf. for example DE 10 2014 104 984 A1).

The performance of the membrane stack in terms of effective depletion of cells and cell debris, and in terms of fouling can be influenced by varying the convective pore size of each membrane layer. For example, the selectable convective pore size of each membrane layer, while keeping the binding capacity constant, can influence depletion as well as fouling. For example, by having a first membrane layer (i.e. the membrane layer facing the tangential-flow channel) with either smaller or larger convective pore size, performance can be influenced. However, membrane layer systems with continuously variable pore size in different arrangements (gradients etc..) can also be combined to a membrane stack to positively influence the overall performance in terms of depletion, fouling, but also dispersion. By using suitable connections, a sterile barrier can be ensured which allows aseptic processing of liquid in combination with appropriate fluid path. The chromatography device can be additionally pre-treated thermally, chemically or by irradiation for this purpose.

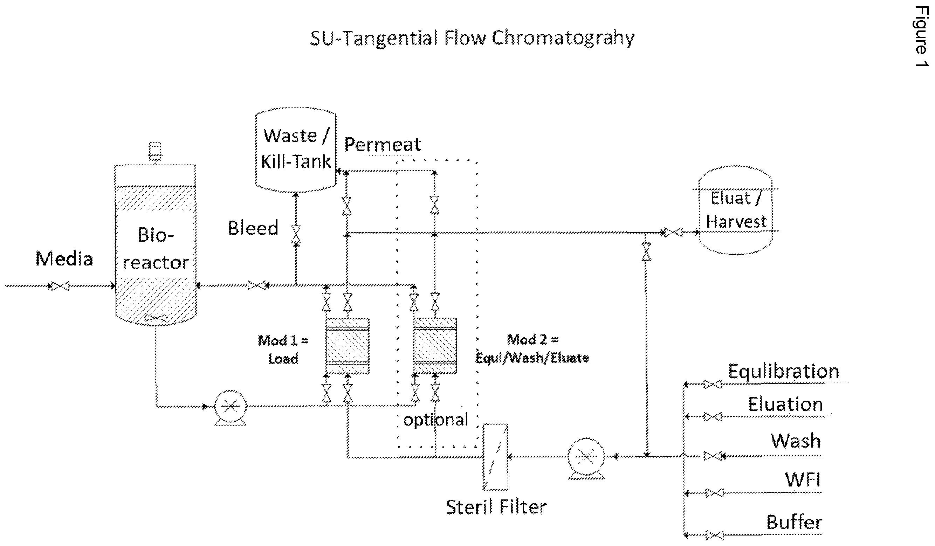

The tangential flow filtration/chromatography system of the present invention can be enlarged by parallelization of two or more tangential flow filtration/chromatography devices as described hereinabove according to the demand (binding capacity, productivity). In this case, the retentate section of the preceding tangential flow filtration/chromatography device is separated from the retentate section of the next tangential flow filtration/chromatography device by another membrane layer. Each retentate section is fluidly (communicatingly) connected to an inlet for the fluid to be filtered and to an outlet for the retentate, and each permeate section is fluidly connected to an outlet for the permeate (cf. Figure 1, square indicated as "optional").

As is known to the skilled person, a dead volume reduced design of a chromatography device as well as dispersion in front of and behind the membrane bed is decisive. The ratio of the at least one membrane volume to the dead volume (= void volume accessible by fluid including pores of the membrane as well as connection geometry) of the tangential flow filtration/chromatography device used in the present invention preferably is < 1:10, and more preferably < 1:5, even more preferably < 1:2.

In one embodiment, the flow filtration/chromatography device used in the present invention is arranged in a recirculation loop connecting the at least one retentate outlet and the at least one fluid inlet of the flow filtration/chromatography device. In this case, fluid from the permeate section can be fed back into the retentate section during loading. This procedure has two advantages: Target molecules or further contaminants that have unwantingly already passed through the membrane bed are returned for reloading. This can help to increase the yield of target molecule or further contaminants (for example in case of unknown or changing binding capacity or poor dispersion). Furthermore, the total capacity of the chromatography bed can be better utilized, since convectively or diffusively less accessible binding sites can also bind target molecule (= increase of residence time). This results in both a higher concentration in the elution and a reduced amount of buffer required to purify a defined amount of target molecule (= fewer cycles required). Moreover, the specific flow rate per filter area can be increased, which means faster loading or shorter process time.

The tangential flow filtration/chromatography system of the present invention may further comprise further constituents used in classical filtration or chromatography system such as pumps, waste tanks, eluate/harvest tanks, etc. which are well-known to those skilled in the art.

The tangential flow filtration/chromatography system of the present invention may be directly connected to a bioreactor, so that the fluid comprising the one or more target molecules, the cells, the cell debris and the further contaminants can be fed inline from the bioreactor into the tangential flow filtration/chromatography system.

According to a preferred embodiment of the present invention, at least the tangential flow filtration/chromatography device used in the present invention is a single-use device. Therefore, costly and time-consuming validation processes of stainless steel systems can be optimized and accelerated in applications in the biopharmaceutical industry. According to a more preferred embodiment of the present invention, further constituents, e.g. the bioreactor, are also of single-use.

The present invention further provides the use of the tangential flow filtration/chromatography systems of the present invention in simultaneously separating one or more target molecules from cells, cell debris and further contaminants. The one or more target molecules, the cells, the cell debris and the further contaminants may e.g. be contained in fermentation broth or any other particulate and impurity containing, non-clarified liquid. The use of the present invention advantageously allows to partially or completely eliminate classical clarification steps such as centrifugation, depth filtration, and also sterile filtration before chromatography.

A further aspect of the present invention relates to methods of simultaneously separating one or more target molecules from cells and cell debris both having a specific minimum particle size and from further contaminants having a specific maximum particle size. Said methods employ the tangential flow filtration/chromatography systems of the present invention, and thus each of the embodiments described above also applies to the methods of the present invention.

A first method of the present invention is a method of simultaneously separating one or more target molecules from cells and cell debris both having a specific minimum particle size and from further contaminants having a specific maximum particle size, the method comprising
(a1) providing a fluid containing the one or more target molecules, the cells, the cell debris and the further contaminants;
(b1) providing the tangential flow filtration/chromatography system of the present invention, which comprises the tangential flow filtration/chromatography device comprising the at least one membrane having chromatographically active centers for selectively binding the one or more target molecules;
(c1) passing the fluid from the at least one fluid inlet to the at least one retentate outlet of the tangential flow filtration/chromatography device tangentially along the surface of the at least one membrane,
wherein the cells and cell debris do not permeate into and through the at least one membrane, the one or more target molecules permeate into the at least one membrane and are bonded to the chromatographically active centers thereof, and the further contaminants permeate into and through the at least one membrane to the permeate section;
(d1) after step (c1) is finished, washing remaining further contaminants out of the tangential flow filtration/chromatography device with a buffer solution; and
(e1) after step (d1) is finished, eluting the one or more target molecules from the chromatographically active centers of the at least one membrane with a different buffer solution to obtain the separated one or more target molecules.

The first method of the present invention is a method in which the cells and the cell debris do not permeate into and through the at least one membrane, the one or more target molecules permeate into the at least one membrane and are selectively bonded to chromatographically active centers thereof, and the further contaminants permeate into and through the at least one membrane to the permeate section.

In step (a1) of the first method, a fluid containing the one or more target molecules, the cells, the cell debris and the further contaminants is provided. The one or more target molecules, the cells, the cell debris and the further contaminants are described in detail hereinabove. The fluid containing the one or more target molecules, the cells, the cell debris and the further contaminants may e.g. be fermentation broth or any other particulate and impurity containing, non-clarified liquid. Providing said fluid may include providing the fluid inline from a bioreactor connected to the tangential flow filtration/chromatography device.

In step (b1) of the first method, the tangential flow filtration/chromatography system of the present invention, which comprises the tangential flow filtration/chromatography device comprising the at least one membrane having chromatographically active centers for selectively binding the one or more target molecules, is provided.

In step (c1) of the first method, the fluid from the at least one fluid inlet is passed to the at least one retentate outlet of the tangential flow filtration/chromatography device tangentially along the surface of the at least one membrane. The cells and cell debris are retained on the upstream side (i.e., in the retentate section of the tangential flow filtration/chromatography) in the fluid stream on the surface of the at least one membrane and are further concentrated there, while the clarified liquid freed from cells and cell debris and containing the one or more target molecules together with the further contaminants permeates into the at least one membrane. The one or more target molecules are selectively bonded to the chromatographically active centers of the at least one membrane, whereas the further contaminants permeate through the at least one membrane to the permeate section.

In step (d1) of the first method after step (c1) is finished, remaining further contaminants (which may be present e.g. non-bonded in the membrane) are washed out of the tangential flow filtration/chromatography device with a buffer solution. The remaining further contaminants are typically washed out into a waste tank.

In step (e1) of the first method after step (d1) is finished, the one or more target molecules are eluted from the chromatographically active centers of the at least one membrane with a different buffer solution to obtain the separated one or more target molecules. The one or more target molecules are typically eluted into a harvest tank. The elution step can be carried out either from the retentate section into the permeate section or from the permeate section into the retentate section.

According to the method of the present invention, it is advantageously possible to handle the cell/cell debris separation step and the chromatography step that would otherwise follow in a next process step, in a single step, which means significant advantages in terms of space and time requirements, as well as cost reduction.

A second method of the present invention is a method of simultaneously separating one or more target molecules from cells and cell debris both having a specific minimum particle size and from further contaminants having a specific maximum particle size, the method comprising
(a2) providing a fluid containing the one or more target molecules, the cells, the cell debris and the further contaminants;
(b2) providing the tangential flow filtration/chromatography of the present invention, which comprises the tangential flow filtration/chromatography device comprising the at least one membrane having chromatographically active centers for selectively binding the further contaminants;
(c2) passing the fluid from the at least one fluid inlet to the at least one retentate outlet of the tangential flow filtration/chromatography device tangentially along the surface of the at least one membrane,
wherein the cells and the cell debris do not permeate into and through the at least one membrane, the further contaminants permeate into the at least one membrane and are bonded to the chromatographically active centers thereof, and the one or more target molecules permeate into and through the at least one membrane to the permeate section to obtain the separated one or more target molecules;
optionally (d2) after step (c2) is finished, washing the remaining one or more target molecules out of the tangential flow filtration/chromatography device with a washing solution to increase the amount of the separated one or more target molecules.

The second method of the present invention is a method in which the cells and the cell debris do not permeate into and through the at least one membrane, the further contaminants permeate into the at least one membrane and are selectively bonded to chromatographically active centers thereof, and the one or more target molecules permeate into and through the at least one membrane to the permeate section to obtain the separated one or more target molecules.

Step (a2) of the second method is identical to step (a1) of the first method of the present invention.

In step (b2) of the second method, the tangential flow filtration/chromatography system of the present invention, which comprises the tangential flow filtration/chromatography device comprising the at least one membrane having chromatographically active centers for selectively binding the further contaminants, is provided.

In step (c2) of the second method, the fluid from the at least one fluid inlet is passed to the at least one retentate outlet of the tangential flow filtration/chromatography device tangentially along the surface of the at least one membrane. The cells and cell debris are retained on the upstream side (i.e., in the retentate section of the tangential flow filtration/chromatography) in the fluid stream on the surface of the at least one membrane and are further concentrated there, while the clarified liquid freed from cells and cell debris and containing the one or more target molecules together with the further contaminants permeates into the at least one membrane. The further contaminants are selectively bonded to the chromatographically active centers of the at least one membrane, whereas the one or more target molecules permeate through the at least one membrane to the permeate section to obtain the separated one or more target molecules.

In an optional step (d2) of the second method after step (c2) is finished, one or more target molecules which may still remain (non-bonded) in the tangential flow filtration/chromatography device may be washed out of the tangential flow filtration/chromatography device with a washing solution to increase the amount of separated one or more target molecules.

The tangential flow filtration mode of the present invention is particularly advantageous due to the composition of the fluid to be separated, because the formation of a cover layer and the associated blocking can be partially or completely avoided by setting suitable process parameters. Thus, the advantages of the tangential filtration and depth filtration principles can be combined and utilized. The large particles (particularly cells and cell debris) are repeatedly washed away in the cross-flow (and may be washed into a waste tank) and, through suitable parameter selection, enable the control or avoidance of a cover layer formation.

The methods of the present invention may further comprise additional steps such as equilibration step(s) before step (c1)/(c2), one or more additional washing steps, stripping step(s) and cleaning/regeneration (e.g. CIP) steps using various washing/cleaning solutions. Such additional steps are well-known to those skilled in the art of chromatography.

The loading of the tangential flow filtration/chromatography device can be done in different ways. One way is the classical chromatography application (Single Pass Tangential Flow Filtration SPTFF; cf. for example WO 2016/033546 A1), in which the permeate in the permeate section is discharged into a waste tank (in the above-described first method of the present invention) or into a harvest tank (in the above-described second method of the present invention).

Alternatively, an appropriate connection of the tangential flow filtration/chromatography device allows to lead back the permeate into the retentate section during loading. That is, the methods of the present invention may further comprise a step of passing the fluid from the permeate section (e.g. through the permeate outlet) into the retentate section (e.g. through the least one fluid inlet), e.g. via a recirculation loop. This procedure has two advantages: Target molecule or further contaminants that have unwantingly already passed through the membrane bed are returned for reloading. This prevents the loss of target molecule or further contaminants (for example in case of unknown or changing binding capacity or poor dispersion). Furthermore, the total capacity of the chromatography bed can be better utilized, since convectively or diffusively less accessible binding sites can also bind target molecule (= increase of residence time). This results in both a higher concentration in the elution and a reduced amount of buffer required to purify a defined amount of target molecule (= fewer cycles required).

A further embodiment of the present invention uses continuous processing of the fluid by a "simulated moving bed" method known to those skilled in the art. In this case, a pseudo-continuous processing of fluid is made possible by a combination of two or more of the tangential flow filtration/chromatography devices described herein.

In order to suppress the formation of a cover layer on the at least one membrane and the possible blocking associated with it by the cells and cell debris, the process control is of decisive importance. For example, the process can be extended by measures known in tangential flow filtration, such as cyclic reversal of the filtration direction (feed reversal) and/or cyclic backflushing, sparging or further cleaning methods such as vibration or ultrasound. This can increase productivity for purification of a classical batch as well as reduce fouling in combination with a perfusion bioreactor for long-term processes.

Directional changes as well as backflushing cause a (partial) detachment of the top layer, especially during loading, and thus favor the permeate performance and cycling stability of the membrane bed. During permeate recirculation, part of the liquid on the permeate section side is directed towards the retentate section during loading. Feed reversal and backflushing can run independently or synchronized with each other. The interval frequency can e.g. be between 30 sec and 15 min. The optimum combination of feed reversal and backflushing as well as their intervals can vary depending on the fluid to be purified.

In sparging, a (inert) gas is used to enhance permeate performance.

The use of the additional measures mentioned above can take place at fixed intervals or can be initiated by appropriate process monitoring (e.g. increase of the pressure drop across the membrane bed above a defined threshold, characterization of the fluid during loading (cell count, temperature, etc.)).

In a further embodiment, the tangential flow filtration/chromatography system provided in step (b1) or (b2) may already be pre-cleaned and in a preserved state.

Due to the system and methods of the present invention, it is advantageously possible in the separation of one or more target molecules from a fermentation broth or any other particulate and impurity containing, non-clarified liquid to handle the cell/cell debris separation step and the chromatography step that would otherwise follow in a next process step, in a single step, which means significant advantages in terms of space and time requirements, as well as cost reduction. Thus, classical clarification steps such as centrifugation, depth filtration, and also sterile filtration before chromatography can be partially or completely eliminated. Moreover, since at least the tangential flow filtration/chromatography device used in the present invention may be a single-use device, costly and time-consuming validation processes of stainless steel systems can be optimized and accelerated in applications in the biopharmaceutical industry.

The Figures show:
Figure 1 shows an exemplary single-use tangential flow filtration/chromatography system in accordance with the present invention.
Figure 2 shows the elution chromatogram of Example 1.

The present invention will be further illustrated in the following example without being limited thereto.

### Example 1 :

A single-use tangential flow filtration/chromatography system according to the present invention as depicted in Figure 1 is provided. The tangential flow filtration/chromatography system comprises a tangential flow filtration/chromatography device comprising a fluid inlet, a retentate outlet, a permeate outlet, and an agarose membrane bed which separates the retentate section from the permeate section in the tangential flow filtration/chromatography device. The retentate outlet is in the retentate section and the permeate outlet is in the permeate section. The agarose membrane bed has chromatographically active centers (protein A) for selectively binding the target molecule, and has a specific convective pore size to block the cells and cell debris both having a specific minimum size larger than the convective pore size from permeating into and through the membrane bed to the permeate section, and to allow the further contaminants having a specific maximum size smaller than or equal to the convective pore size to permeate into and through the membrane bed to the permeate section.

Flow fittings are used in the retentate section and in the permeate section to keep the channels of the membrane bed open in the presence of cells/cell debris.

The following filtration/chromatography protocol is employed:

| Step | Buffer | Flow rate [MV/min] | volume [MV] |
|---|---|---|---|
| Equillibration (EQ) | 1 M Tris HCL | 10 | 5 |
| Fluid feed/Loading | | 5 | depending on DBC |
| Washing | 1x PBS | 40 | 12 |
| Elution | 0.1 M AcOH; 0.15 M NaCl | 2.5 | 12 |
| CIP | 0.2 M NaOH | 5 | 20 |
| Re-equillibration (ReEQ) | 1 M Tris HCL | 10 | 20 |

In the EQ phase, the EQ buffer enters the tangential flow filtration/chromatography device and exits the tangential flow filtration/chromatography device into a waste cup.

The loading phase (B) is performed in cross-flow mode, which means liquid recirculation. This recirculation is performed in "reverse mode", which means that the loading direction (change of direction feed - retentate) is constantly changed. The permeate leaving the tangential flow filtration/chromatography device is collected in a beaker on a balance to measure the permeate volume. The permeate can also be used for periodic permeate backwashing of the membrane bed during loading of the membranes. UV levels of the permeate are measured to detect product breakthrough. In the wash phase, wash buffer is pumped backward or forward through the tangential flow filtration/chromatography device into the waste tank in the feed line or into the feed tank. In the elution phase (D), elution buffer enters the tangential flow filtration/chromatography device and exits through the elution line. To detect the elution peak, the UV values of the elution front are measured. Starting from a certain UV value (e.g. 0.05 CU) at the beginning of the elution peak, the eluate is collected in a separate elution cup until this value is reached again at the end of the peak. In the CIP (cleaning-in-place) and ReEQ phases, the CIP or ReEQ buffer is fed backwards into the tangential flow filtration/chromatography device and exits the flow cell into the waste container in the feed line.

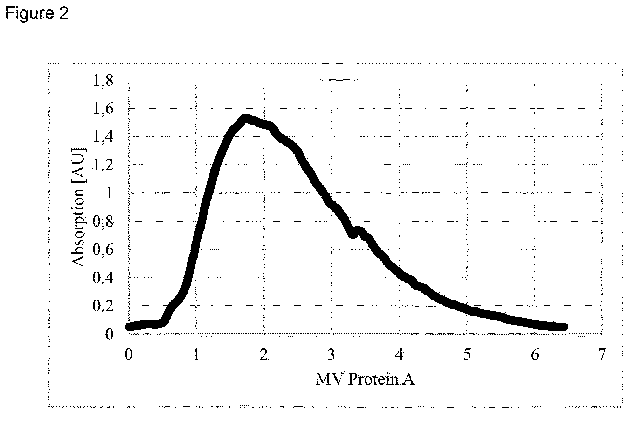

The following Table summarizes the applied settings of the example performed with a yeast model suspension mixed with Cutaquig - a polyclonal antibody - as the target molecule. The course of the process is shown in the elution chromatogram of Figure 2. The elution peak corresponds to a concentration of 4.65 mg/mL (measured on the Lunatic Chip Reader) or 4.49 mg/mL (integrated area).

| Setting | Value |
|---|---|
| Titer Permeate backwashing | 4 g/L |
| | every 5 s for 0.5 s |
| Amount of permeate | 143.5 mL (corresponds to a loading at which the DBC10% is reached; DBC10% assumed: 50 mg/mL MV protein A at a retention time of 1 min) |
| Yeast model suspension | 200 mL |

## Claims

1. A tangential flow filtration/chromatography system for simultaneously separating one or more target molecules from cells, cell debris and further contaminants, the flow filtration/chromatography system comprising
a tangential flow filtration/chromatography device comprising at least one fluid inlet, at least one retentate outlet, at least one permeate outlet, and at least one membrane which separates a retentate section from a permeate section in the tangential flow filtration/chromatography device, the at least one retentate outlet being in the retentate section and the least one permeate outlet being in the permeate section,
wherein a flow from the at least one fluid inlet to the at least one retentate outlet is guided in the tangential flow filtration/chromatography device tangentially along the surface of the at least one membrane,
wherein the at least one membrane has chromatographically active centers for selectively binding the one or more target molecules or for selectively binding the further contaminants, and has convective pores which are flow through macropores providing flow passages or channels through the membrane and having a specific convective pore size;
wherein the at least one membrane comprises a polymer network material-based self-supporting bi-continuous separation matrix comprising a first continuous phase and a second continuous phase, the at least one membrane optionally comprising a third inert phase, wherein the first continuous phase is a portion of the matrix which is formed by a self-gelled polysaccharide and wherein the second continuous phase is a portion of the matrix which defines the convective pores which are non-cylindrical large diameter continuously connected convective pores in-between the first continuous phase, wherein the non-cylindrical large diameter continuously connected convective pores have a median pore diameter of from 0.10 µm to 30 µm, and wherein the third inert phase does not constitute the structure of the second continuous phase.

2. Use of the tangential flow filtration/chromatography system according to claim 1 in simultaneously separating one or more target molecules from cells, cell debris and further contaminants.

3. A method of simultaneously separating one or more target molecules from cells and cell debris both having a specific minimum particle size and from further contaminants having a specific maximum particle size, the method comprising
(a1) providing a fluid containing the one or more target molecules, the cells, the cell debris and the further contaminants;
(b1) providing the tangential flow filtration/chromatography system according to claim 1, in which the at least one membrane has chromatographically active centers for selectively binding the one or more target molecules;
(c1) passing the fluid from the at least one fluid inlet to the at least one retentate outlet of the tangential flow filtration/chromatography device tangentially along the surface of the at least one membrane,
wherein the cells and cell debris do not permeate into and through the at least one membrane, the one or more target molecules permeate into the at least one membrane and are bonded to the chromatographically active centers thereof, and the further contaminants permeate into and through the at least one membrane to the permeate section;
(d1) after step (c1) is finished, washing remaining further contaminants out of the tangential flow filtration/chromatography device with a buffer solution; and (e1) after step (d1) is finished, eluting the one or more target molecules from the chromatographically active centers of the at least one membrane with a different buffer solution to obtain the separated one or more target molecules.

4. A method of simultaneously separating one or more target molecules from cells and cell debris both having a specific minimum particle size and from further contaminants having a specific maximum particle size, the method comprising
(a2) providing a fluid containing the one or more target molecules, the cells, the cell debris and the further contaminants;
(b2) providing the tangential flow filtration/chromatography system according to claim 1, in which the at least one membrane has chromatographically active centers for selectively binding the further contaminants,
(c2) passing the fluid from the at least one fluid inlet to the at least one retentate outlet of the tangential flow filtration/chromatography device tangentially along the surface of the at least one membrane,
wherein the cells and the cell debris do not permeate into and through the at least one membrane, the further contaminants permeate into the at least one membrane and are bonded to the chromatographically active centers thereof, and the one or more target molecules permeate into and through the at least one membrane to the permeate section to obtain the separated one or more target molecules;
optionally (d2) after step (c2) is finished, washing the remaining one or more target molecules out of the tangential flow filtration/chromatography device with a washing solution to increase the amount of the separated one or more target molecules.

5. The system, use or method according to any one of claims 1 to 4,
wherein the self-gelled polysaccharide is one or more selected from the group consisting of agarose, agar, agaropectin, kappa-carrageenan, iota-carrageenan, lambda-carrageenan, gellan gum, amylose, curdlan, alginate and rhamsan gum, and is most preferably agarose.

6. The system, use or method according to any one of claims 1 to 5,
wherein a volume ratio of the self-gelled polysaccharide to the total volume of the at least one membrane including the third inert phase is at least 30 vol%.

7. The system, use or method according to any one of claims 1 to 6,
wherein the tangential flow filtration/chromatography device further comprises a microfiltration membrane or ultrafiltration membrane in the retentate section and/or the permeate section, the microfiltration membrane or ultrafiltration membrane having a preferred mean pore size diameter of from 0.10 to 10 µm.

8. The system, use or method according to any one of claims 1 to 7,
wherein the tangential flow filtration/chromatography system comprises two or more layers of the membrane constituting a membrane bed.

9. The system, use or method according to claim 8,
wherein the membrane bed has a thickness of from 0.10 to 40 mm.

10. The system, use or method according to claim 8 or 9,
wherein the membrane bed further comprises, between the membranes, one or more inert layers having no chromatographically active centers.

11. The system, use or method according to any one of claims 1 to 10,
wherein the tangential flow filtration/chromatography device is arranged in a recirculation loop connecting the at least one retentate outlet and the at least one fluid inlet of the tangential flow filtration/chromatography device.

12. The system, use or method according to any one of claims 1 to 11,
wherein the tangential flow filtration/chromatography device is a single-use device.

## Patentansprüche

1. Tangentialfluss-Filtrations-/Chromatographiesystem zur gleichzeitigen Abtrennung eines oder mehrerer Zielmoleküle von Zellen, Zelltrümmern und weiteren Verunreinigungen, wobei das Filtrations-/Chromatographiesystem umfasst:
eine Tangentialfluss-Filtrations-/Chromatographievorrichtung, umfassend mindestens einen Fluideinlass, mindestens einen Retentatauslass, mindestens einen Permeatauslass und mindestens eine Membran, die einen Retentatabschnitt von einem Permeatabschnitt in der Tangentialfluss-Filtrations-/Chromatographievorrichtung trennt, wobei sich der mindestens eine Retentatauslass in dem Retentatabschnitt und der mindestens eine Permeatauslass in dem Permeatabschnitt befindet,
wobei ein Strom von dem mindestens einen Fluideinlass zu dem mindestens einen Retentatauslass in der Tangentialfluss-Filtrations-/Chromatographievorrichtung tangential entlang der Oberfläche der mindestens einen Membran geführt wird,
wobei die mindestens eine Membran chromatographisch aktive Zentren zum selektiven Binden des einen oder der mehreren Zielmoleküle oder zum selektiven Binden der weiteren Verunreinigungen aufweist und konvektive Poren aufweist, die durchströmte Makroporen sind, die Strömungsdurchgänge oder -kanäle durch die Membran bereitstellen und eine spezifische konvektive Porengröße aufweisen;
wobei die mindestens eine Membran eine selbsttragende bikontinuierliche Trennmatrix auf Basis eines Polymernetzwerkmaterials umfasst, die eine erste kontinuierliche Phase und eine zweite kontinuierliche Phase umfasst, wobei die mindestens eine Membran gegebenenfalls eine dritte inerte Phase umfasst, wobei die erste kontinuierliche Phase ein Teil der Matrix ist, der durch ein selbstgeliertes Polysaccharid gebildet ist, und wobei die zweite kontinuierliche Phase ein Teil der Matrix ist, der die konvektiven Poren definiert, die nicht zylindrische, kontinuierlich verbundene konvektive Poren mit großem Durchmesser zwischen der ersten kontinuierlichen Phase sind, wobei die nicht zylindrischen, kontinuierlich verbundenen konvektiven Poren mit großem Durchmesser einen medianen Porendurchmesser von 0,10 µm bis 30 µm aufweisen, und wobei die dritte inerte Phase nicht die Struktur der zweiten kontinuierlichen Phase bildet.

2. Verwendung des Tangentialfluss-Filtrations-/Chromatographiesystems nach Anspruch 1 zur gleichzeitigen Abtrennung eines oder mehrerer Zielmoleküle von Zellen, Zelltrümmern und weiteren Verunreinigungen.

3. Verfahren zur gleichzeitigen Abtrennung eines oder mehrerer Zielmoleküle von Zellen und Zelltrümmern, die beide eine spezifische minimale Partikelgröße aufweisen, und von weiteren Verunreinigungen, die eine spezifische maximale Partikelgröße aufweisen, wobei das Verfahren umfasst:
(a1) Bereitstellen eines Fluids, das das eine oder die mehreren Zielmoleküle, die Zellen, die Zelltrümmer und die weiteren Verunreinigungen enthält;
(b1) Bereitstellen des Tangentialfluss-Filtrations-/Chromatographiesystems nach Anspruch 1, bei dem die mindestens eine Membran chromatographisch aktive Zentren zum selektiven Binden des einen oder der mehreren Zielmoleküle aufweist;
(c1) Leiten des Fluids von dem mindestens einen Fluideinlass zu dem mindestens einen Retentatauslass der Tangentialfluss-Filtrations-/Chromatographievorrichtung tangential entlang der Oberfläche der mindestens einen Membran,
wobei die Zellen und Zelltrümmer nicht in die mindestens eine Membran eindringen und nicht durch diese hindurchtreten, das eine oder die mehreren Zielmoleküle in die mindestens eine Membran eindringen und an deren chromatographisch aktive Zentren gebunden werden, und die weiteren Verunreinigungen in die mindestens eine Membran eindringen und durch diese hindurch in den Permeatabschnitt treten;
(d1) nachdem Schritt (c1) beendet ist, Auswaschen verbleibender weiterer Verunreinigungen aus der Tangentialfluss-Filtrations-/Chromatographievorrichtung mit einer Pufferlösung; und
(e1) nachdem Schritt (d1) beendet ist, Eluieren des einen oder der mehreren Zielmoleküle von den chromatographisch aktiven Zentren der mindestens einen Membran mit einer anderen Pufferlösung, um das eine oder die mehreren abgetrennten Zielmoleküle zu erhalten.

4. Verfahren zur gleichzeitigen Abtrennung eines oder mehrerer Zielmoleküle von Zellen und Zelltrümmern, die beide eine spezifische minimale Partikelgröße aufweisen, und von weiteren Verunreinigungen, die eine spezifische maximale Partikelgröße aufweisen, wobei das Verfahren umfasst:
(a2) Bereitstellen eines Fluids, das das eine oder die mehreren Zielmoleküle, die Zellen, die Zelltrümmer und die weiteren Verunreinigungen enthält;
(b2) Bereitstellen des Tangentialfluss-Filtrations-/Chromatographiesystems nach Anspruch 1, bei dem die mindestens eine Membran chromatographisch aktive Zentren zum selektiven Binden der weiteren Verunreinigungen aufweist,
(c2) Leiten des Fluids von dem mindestens einen Fluideinlass zu dem mindestens einen Retentatauslass der Tangentialfluss-Filtrations-/Chromatographievorrichtung tangential entlang der Oberfläche der mindestens einen Membran,
wobei die Zellen und die Zelltrümmer nicht in die mindestens eine Membran eindringen und nicht durch diese hindurchtreten, die weiteren Verunreinigungen in die mindestens eine Membran eindringen und an deren chromatographisch aktive Zentren gebunden werden, und das eine oder die mehreren Zielmoleküle in die mindestens eine Membran eindringen und durch diese hindurch in den Permeatabschnitt treten, um das eine oder die mehreren abgetrennten Zielmoleküle zu erhalten;
(d2) gegebenenfalls, nachdem Schritt (c2) beendet ist, Auswaschen des einen oder der mehreren verbleibenden Zielmoleküle aus der Tangentialfluss-Filtrations-/Chromatographievorrichtung mit einer Waschlösung, um die Menge des einen oder der mehreren abgetrennten Zielmoleküle zu erhöhen.

5. System, Verwendung oder Verfahren nach einem der Ansprüche 1 bis 4,
wobei das selbstgelierte Polysaccharid eines oder mehrere ist, ausgewählt aus der Gruppe bestehend aus Agarose, Agar, Agaropectin, kappa-Carrageenan, iota-Carrageenan, lambda-Carrageenan, Gellangummi, Amylose, Curdlan, Alginat und Rhamsangummi, und am meisten bevorzugt Agarose ist.

6. System, Verwendung oder Verfahren nach einem der Ansprüche 1 bis 5,
wobei ein Volumenverhältnis des selbstgelierten Polysaccharids zum Gesamtvolumen der mindestens einen Membran einschließlich der dritten inerten Phase mindestens 30 Vol.-% beträgt.

7. System, Verwendung oder Verfahren nach einem der Ansprüche 1 bis 6,
wobei die Tangentialfluss-Filtrations-/Chromatographievorrichtung ferner eine Mikrofiltrationsmembran oder Ultrafiltrationsmembran in dem Retentatabschnitt und/oder dem Permeatabschnitt umfasst, wobei die Mikrofiltrationsmembran oder Ultrafiltrationsmembran einen bevorzugten mittleren Porengrößendurchmesser von 0,10 bis 10 µm aufweist.

8. System, Verwendung oder Verfahren nach einem der Ansprüche 1 bis 7,
wobei das Tangentialfluss-Filtrations-/Chromatographiesystem zwei oder mehr Schichten der Membran umfasst, die ein Membranbett bilden.

9. System, Verwendung oder Verfahren nach Anspruch 8,
wobei das Membranbett eine Dicke von 0,10 bis 40 mm aufweist.

10. System, Verwendung oder Verfahren nach Anspruch 8 oder 9,
wobei das Membranbett ferner zwischen den Membranen eine oder mehrere inerte Schichten umfasst, die keine chromatographisch aktiven Zentren aufweisen.

11. System, Verwendung oder Verfahren nach einem der Ansprüche 1 bis 10,
wobei die Tangentialfluss-Filtrations-/Chromatographievorrichtung in einer Rezirkulationsschleife angeordnet ist, die den mindestens einen Retentatauslass und den mindestens einen Fluideinlass der Tangentialfluss-Filtrations-/Chromatographievorrichtung verbindet.

12. System, Verwendung oder Verfahren nach einem der Ansprüche 1 bis 11,
wobei die Tangentialfluss-Filtrations-/Chromatographievorrichtung eine Einwegvorrichtung ist.

## Revendications

1. Système de filtration/chromatographie à flux tangentiel pour la séparation simultanée d'une ou plusieurs molécules cibles de cellules, de débris cellulaires et d'autres contaminants, le système de filtration/chromatographie comprenant
un dispositif de filtration/chromatographie à flux tangentiel comprenant au moins une entrée de fluide, au moins une sortie de rétentat, au moins une sortie de perméat et au moins une membrane qui sépare une section de rétentat d'une section de perméat dans le dispositif de filtration/chromatographie à flux tangentiel, l'au moins une sortie de rétentat se trouvant dans la section de rétentat et l'au moins une sortie de perméat se trouvant dans la section de perméat,
dans lequel un écoulement de l'au moins une entrée de fluide vers l'au moins une sortie de rétentat est guidé dans le dispositif de filtration/chromatographie à flux tangentiel tangentiellement le long de la surface de l'au moins une membrane,
dans lequel l'au moins une membrane présente des centres chromatographiquement actifs pour lier sélectivement la ou les molécules cibles ou pour lier sélectivement les autres contaminants, et présente des pores convectifs qui sont des macropores traversants fournissant des passages ou canaux d'écoulement à travers la membrane et présentant une taille de pore convectif spécifique ;
dans lequel l'au moins une membrane comprend une matrice de séparation bicontinue autoportante à base d'un matériau à réseau polymère comprenant une première phase continue et une deuxième phase continue, l'au moins une membrane comprenant facultativement une troisième phase inerte, dans lequel la première phase continue est une partie de la matrice qui est formée par un polysaccharide autogélifié et dans lequel la deuxième phase continue est une partie de la matrice qui définit les pores convectifs qui sont des pores convectifs non cylindriques de grand diamètre reliés de manière continue entre la première phase continue, dans lequel les pores convectifs non cylindriques de grand diamètre reliés de manière continue présentent un diamètre de pore médian de 0,10 µm à 30 µm, et dans lequel la troisième phase inerte ne constitue pas la structure de la deuxième phase continue.

2. Utilisation du système de filtration/chromatographie à flux tangentiel selon la revendication 1 pour la séparation simultanée d'une ou plusieurs molécules cibles de cellules, de débris cellulaires et d'autres contaminants.

3. Procédé de séparation simultanée d'une ou plusieurs molécules cibles, d'une part de cellules et de débris cellulaires présentant tous deux une taille de particule minimale spécifique, et d'autre part d'autres contaminants présentant une taille de particule maximale spécifique, le procédé comprenant
(a1) la fourniture d'un fluide contenant la ou les molécules cibles, les cellules, les débris cellulaires et les autres contaminants ;
(b1) la fourniture du système de filtration/chromatographie à flux tangentiel selon la revendication 1, dans lequel l'au moins une membrane présente des centres chromatographiquement actifs pour lier sélectivement la ou les molécules cibles ;
(c1) le passage du fluide de l'au moins une entrée de fluide vers l'au moins une sortie de rétentat du dispositif de filtration/chromatographie à flux tangentiel tangentiellement le long de la surface de l'au moins une membrane,
dans lequel les cellules et les débris cellulaires ne pénètrent pas dans l'au moins une membrane ni ne la traversent, la ou les molécules cibles pénètrent dans l'au moins une membrane et sont liées aux centres chromatographiquement actifs de celle-ci, et les autres contaminants pénètrent dans l'au moins une membrane et la traversent jusqu'à la section de perméat ;
(d1) après que l'étape (c1) est terminée, le lavage des autres contaminants restants hors du dispositif de filtration/chromatographie à flux tangentiel avec une solution tampon ; et
(e1) après que l'étape (d1) est terminée, l'élution de la ou des molécules cibles des centres chromatographiquement actifs de l'au moins une membrane avec une solution tampon différente pour obtenir la ou les molécules cibles séparées.

4. Procédé de séparation simultanée d'une ou plusieurs molécules cibles, d'une part de cellules et de débris cellulaires présentant tous deux une taille de particule minimale spécifique, et d'autre part d'autres contaminants présentant une taille de particule maximale spécifique, le procédé comprenant
(a2) la fourniture d'un fluide contenant la ou les molécules cibles, les cellules, les débris cellulaires et les autres contaminants ;
(b2) la fourniture du système de filtration/chromatographie à flux tangentiel selon la revendication 1, dans lequel l'au moins une membrane présente des centres chromatographiquement actifs pour lier sélectivement les autres contaminants,
(c2) le passage du fluide de l'au moins une entrée de fluide vers l'au moins une sortie de rétentat du dispositif de filtration/chromatographie à flux tangentiel tangentiellement le long de la surface de l'au moins une membrane,
dans lequel les cellules et les débris cellulaires ne pénètrent pas dans l'au moins une membrane ni ne la traversent, les autres contaminants pénètrent dans l'au moins une membrane et sont liés aux centres chromatographiquement actifs de celle-ci, et la ou les molécules cibles pénètrent dans l'au moins une membrane et la traversent jusqu'à la section de perméat pour obtenir la ou les molécules cibles séparées ;
(d2) facultativement, après que l'étape (c2) est terminée, le lavage de la ou des molécules cibles restantes hors du dispositif de filtration/chromatographie à flux tangentiel avec une solution de lavage pour augmenter la quantité de la ou des molécules cibles séparées.

5. Système, utilisation ou procédé selon l'une quelconque des revendications 1 à 4,
dans lequel le polysaccharide autogélifié est un ou plusieurs choisis dans le groupe constitué de l'agarose, de l'agar, de l'agaropectine, du kappa-carraghénane, de l'iota-carraghénane, du lambda-carraghénane, de la gomme gellane, de l'amylose, du curdlane, de l'alginate et de la gomme rhamsane, et est le plus préférablement l'agarose.

6. Système, utilisation ou procédé selon l'une quelconque des revendications 1 à 5,
dans lequel un rapport volumique du polysaccharide autogélifié au volume total de l'au moins une membrane incluant la troisième phase inerte est d'au moins 30 % vol.

7. Système, utilisation ou procédé selon l'une quelconque des revendications 1 à 6,
dans lequel le dispositif de filtration/chromatographie à flux tangentiel comprend en outre une membrane de microfiltration ou une membrane d'ultrafiltration dans la section de rétentat et/ou la section de perméat, la membrane de microfiltration ou la membrane d'ultrafiltration présentant un diamètre moyen de taille de pore préféré de 0,10 à 10 µm.

8. Système, utilisation ou procédé selon l'une quelconque des revendications 1 à 7,
dans lequel le système de filtration/chromatographie à flux tangentiel comprend deux couches ou plus de la membrane constituant un lit membranaire.

9. Système, utilisation ou procédé selon la revendication 8,
dans lequel le lit membranaire présente une épaisseur de 0,10 à 40 mm.

10. Système, utilisation ou procédé selon la revendication 8 ou 9,
dans lequel le lit membranaire comprend en outre, entre les membranes, une ou plusieurs couches inertes ne présentant pas de centres chromatographiquement actifs.

11. Système, utilisation ou procédé selon l'une quelconque des revendications 1 à 10,
dans lequel le dispositif de filtration/chromatographie à flux tangentiel est agencé dans une boucle de recirculation reliant l'au moins une sortie de rétentat et l'au moins une entrée de fluide du dispositif de filtration/chromatographie à flux tangentiel.

12. Système, utilisation ou procédé selon l'une quelconque des revendications 1 à 11,
dans lequel le dispositif de filtration/chromatographie à flux tangentiel est un dispositif à usage unique.
